# FASCICULE DE BREVET EUROPEEN

(11) **EP 1 920 833 B1**
(45) Date de publication et mention de la délivrance du brevet: **20.04.2011**
(21) Numéro de dépôt: 07291222.3
(22) Date de dépôt: 08.10.2007
(51) Int. Cl.: B01J 23/42, B01J 23/46, B01J 23/89, B01J 37/02, B01J 37/16, C07C 5/31

(54) **Catalyseur bimetallique à base de platine et de rhodium utilisé pour l'ouverture de composés cycliques**
Bimetall-Katalysator auf der Basis von Platin und Rhodium zur Ringöffnung von zyklischen Verbindungen
Bimetallic catalyst based on platinum and rhodium used for opening ring compounds

(30) Priorité: 07.11.2006 FR 0609766
(43) Date de publication de la demande: 14.05.2008
(73) Titulaire: IFP Energies nouvelles, 92852 Rueil-Malmaison Cedex (FR)
(72) Inventeur: Mabilon, Gil, 69009 Lyon (FR); Marecot, Patrice, 86130 Saint Georges les Baillargeaux (FR); Uzio, Denis, 69220 Belleville (FR); Especel, Catherine, 86180 Buxerolles (FR); Epron, Florence, 86170 Neuville de Poitou (FR)

(56) Documents cités:
- EP-A1- 0 875 288
- GB-A- 2 211 756
- DEES M J ET AL: "Effect of platinum modification by alloying and sulfur on the hydrogenolysis and aromatization of methylcyclopentane" APPL CATAL; APPLIED CATALYSIS SEP 1990, vol. 64, no. 1-2, septembre 1990 (1990-09), pages 279-295, XP002436539
- F GARIN, G MAIRE: "Possible surface intermediates in alkane reactions on metallic catalysts" ACCOUNTS OF CHEMICAL RESEARCH, [Online] vol. 22, 1989, pages 100-106, XP002436538 USACS, WASHINGTON, DC. Extrait de l'Internet: URL:http://pubs.acs.org/cgi-bin/abstract.c gi/achre4/1989/22/i03/f-pdf/f_ar00159a003. pdf?sessid=6006l3> [extrait le 2007-06-06]

## Description

### Domaine de l'invention

La présente invention se rapporte au domaine des catalyseurs utilisés dans les procédés réalisant l'ouverture sélective des composés naphténiques en vue de l'amélioration de la qualité des essences. Plus précisément, il s'agit d'un catalyseur métallique destiné à l'ouverture sélective du méthylcyclopentane et/ou du méthylcyclohexane.

### Etat de la technique antérieure

Devant les réglementations pour la protection de l'environnement de plus en plus strictes, il est nécessaire de produire des essences de plus en plus propres. En particulier, les essences, aujourd'hui employées comme carburants automobiles, doivent être appauvries en composés aromatiques et enrichies en paraffines, notamment en paraffines ramifiées ayant un haut indice d'octane. La production de telles essences suppose l'hydrogénation totale des composés aromatiques en composés naphténiques puis l'ouverture sélective desdits composés naphténiques en paraffines. La réaction d'ouverture sélective des composés naphténiques en paraffines a déjà été décrite dans l'art antérieur. En particulier, le brevet US 5.763.731 enseigne d'utiliser un catalyseur contenant de l'iridium, du ruthénium et/ou du rhodium pour réaliser l'ouverture de cycles naphténiques, notamment de cycles à 6 atomes de carbone contenant au moins un substituent ayant au moins 3 atomes de carbone. Le document US 5.763.731 enseigne que les meilleurs résultats catalytiques en termes de conversion et de sélectivité en ouverture de cycle envers les produits désirés sont obtenus en présence d'un catalyseur à base d'iridium. L'inconvénient majeur des catalyseurs à base d'iridium est la très faible quantité de ce métal à l'état naturel. Ce manque de disponibilité naturelle rend incompatible la fabrication en gros tonnage de tels catalyseurs à base d'iridium pour leur mise en oeuvre, à l'échelle industrielle, dans des unités d'ouverture de cycles naphténiques. Aussi, la présente invention se propose de fournir un catalyseur métallique pour la mise en oeuvre d'un procédé d'ouverture de cycles naphténiques, démontrant des performances catalytiques voisines de celles obtenues avec un catalyseur à base d'iridium en alternative aux catalyseurs connus de l'art antérieur.

### Description de l'invention

La présente invention concerne un catalyseur comprenant au moins un support poreux, au moins du platine et au moins du rhodium, ledit catalyseur ayant été préparé selon un procédé comprenant au moins :
a) l'imprégnation du support par au moins une solution contenant un précurseur de platine,
b) l'activation sous atmosphère neutre ou oxydante à une température comprise entre 120 et 800°C,
c) l'activation en milieu réducteur à une température comprise entre 0 et 800°C,
d1) l'imprégnation par une solution aqueuse et d2) le traitement par au moins un composé donneur d'hydrogène à une température inférieure à 200 °C, l'ordre de réalisation des étapes d1) et d2) étant indifférent dès lors que lesdites étapes sont réalisées à la suite de ladite étape c) et préalablement à l'étape e) qui suit,
e) l'imprégnation du support, déjà imprégné par du platine, par au moins une solution contenant un précurseur de rhodium,
f) l'activation sous atmosphère neutre, réductrice ou oxydante à une température comprise entre 100 et 800°C.

Le support poreux employé dans le catalyseur selon l'invention est choisi parmi les oxydes de titane, les oxydes de zirconium, les alumines, les silices-alumines, les alumines chlorées, les alumines promues par du fluor, du silicium, du zirconium ou du titane. Les alumines peuvent être choisies parmi l'alumine rô, l'alumine gamma, alumine êta, l'alumine delta, l'alumine thêta, l'alumine kappa, l'alumine alpha et leurs mélanges. De manière préférée, le support poreux est choisi parmi les alumines et les alumines chlorées et de manière très préférée, ledit support poreux est une alumine chlorée.

Une alumine chlorée est généralement préparée par imprégnation d'une alumine par de l'acide chlorhydrique. L'imprégnation peut être réalisée selon les différentes techniques connues de l'homme de l'art telle que l'imprégnation "à sec" ou l'imprégnation par excès de solution. Dans l'imprégnation "à sec", une solution d'acide chlorhydrique est mise en contact avec de l'alumine par exemple par aspersion de la solution sur des billes ou des extrudés d'alumine. Le volume de solution chlorhydrique est choisi égal au volume poreux de l'alumine de sorte que l'alumine imprégnée garde un aspect pratiquement sec même à la fin de l'imprégnation. La concentration en acide chlorhydrique dans la solution est choisie en considérant que tout le chlore est fixé par l'alumine. Dans l'imprégnation par excès de solution, une solution d'acide chlorhydrique est mise en contact avec l'alumine, le volume de solution étant nettement supérieur au volume poreux de l'alumine. Il se produit un échange entre les ions chlore en solution et la surface de l'alumine. La quantité de chlore fixée par l'alumine est fonction des conditions d'échange : concentration en chlore dans la solution, température, durée de l'échange. La concentration préférée en chlore dans l'alumine dépend de la nature de l'alumine et de sa surface spécifique. Une concentration massique comprise entre 0,5 et 2 % poids de chlore dans l'alumine est généralement choisie pour la chloration d'une alumine gamma de surface spécifique comprise entre 150 et 300 m²/g.

Selon l'invention, ledit support poreux se présente avantageusement sous forme de billes, d'extrudés, de pastilles ou de poudre. De manière très avantageuse, ledit support se présente sous forme de billes ou d'extrudés. Le volume poreux du support est compris entre 0,1 et 1,5 cm³/g, de manière préférée compris entre 0,4 et 0,8 cm³/g. Ce volume poreux résulte de la présence essentiellement de mésopores de diamètre de pores compris entre 3 et 20 nm. Ledit support poreux présente une surface spécifique comprise avantageusement entre 150 et 300 m²/g.

Le catalyseur selon l'invention contient un second métal du groupe VIII de la classification périodique des éléments différent du platine et de l'iridium. Ledit second métal est le rhodium.

Le catalyseur selon l'invention contient également avantageusement du chlore.

Le catalyseur selon l'invention contient plus particulièrement :
- au moins du platine présent dans ledit catalyseur en une teneur pondérale représentant de 0,1 à 4 % poids, préférentiellement de 0,2 à 2 % poids et très préférentiellement de 0,3 à 1,5 % poids du catalyseur,
- au moins du rhodium, présent dans ledit catalyseur en une teneur pondérale représentant de 0,05 à 3 % poids, préférentiellement de 0,1 à 1 % poids et très préférentiellement de 0,2 à 0,6 % poids du catalyseur,
- éventuellement du chlore présent dans ledit catalyseur en une teneur pondérale représentant de 0,1 à 3 % poids, préférentiellement de 0,2 à 2 % poids et très préférentiellement de 0,3 à 1,5 % poids du catalyseur,
- au moins un support poreux assurant le complément à 100% poids dans le catalyseur.

L'étape a) du procédé de préparation du catalyseur selon l'invention consiste à mettre au moins un précurseur de platine en solution dans un solvant organique ou inorganique. L'imprégnation peut être effectuée avec un volume de solution compris entre le dixième du volume poreux du support et cent fois le volume poreux dudit support. L'Homme du métier adapte le procédé à la morphologie dudit support poreux selon qu'il est sous forme de billes, de poudre, d'extrudés ou de pastilles. Pour la mise en oeuvre de ladite étape a), le précurseur de platine est notamment choisi parmi l'acide chloroplatinique, l'acide hydroxyplatinique, le dinitroso-diammino platine, le nitrate ou le chlorure de platine tétrammine, de préférence il s'agit de l'acide chloroplatinique. La concentration du précurseur de platine dans la solution est choisie telle que, à l'issue du procédé de préparation du catalyseur selon l'invention, la concentration en platine dans ledit catalyseur, exprimée en poids par rapport audit catalyseur, est comprise entre 0,1 et 4 % poids, préférentiellement entre 0,2 et 2 % poids et très préférentiellement entre 0,3 et 1,5 % poids. La solution contenant le précurseur de platine peut, si nécessaire, contenir des ions hydrogène, ammonium, chlorures, nitrates destinés à modifier les conditions d'acido-basicité du milieu et/ou modifier le nombre de sites d'échanges du support poreux.

La température d'imprégnation du précurseur du platine est en général comprise entre 0 et 100°C, de préférence entre 10 et 50°C. La durée d'imprégnation est en général comprise entre 1 minute et 10 heures, de préférence entre 2 minutes et 4 heures. Selon l'étape b) du procédé de préparation du catalyseur selon l'invention, le support poreux, imprégné de platine, est activé thermiquement à une température comprise entre 120 et 800°C de préférence entre 300 et 600°C. L'atmosphère de traitement est oxydante ou neutre. Elle peut par exemple être constituée d'azote, d'argon, d'air, d'oxygène, de gaz de combustion de mélanges d'air et d'hydrocarbures de richesse inférieure ou égale à 1 et de leurs mélanges, de préférence il s'agit d'air. La durée du traitement est généralement comprise entre 30 minutes et 30 heures, de préférence entre 1 et 6 heures.

Le support ainsi traité selon ladite étape b) est soumis à un traitement en milieu réducteur à une température comprise entre 0 et 800°C de préférence entre 100 et 550°C conformément à l'étape c) du procédé de préparation du catalyseur selon l'invention. Ledit milieu réducteur contient des composés purs ou des mélanges de composés tels que par exemple l'hydrogène, le monoxyde de carbone, les hydrocarbures aliphatiques ou aromatiques, saturés ou insaturés, tels que le méthane, l'éthane, le propane, le butane, l'éthylène, le propylène, l'acétylène, le benzène ou le toluène, les acides carboxyliques tels que l'acide formique ou l'acide acétique, les aldéhydes tels que le formaldéhyde ou l'acétaldéhyde, les alcools tels que le méthanol, l'éthanol ou le propanol, les polyols tels que l'éthylène glycol ou le propylène glycol, les amines tels que la méthylamine ou l'éthylamine, l'urée, l'acide isocyanique, l'hydroxylamine, l'hydrazine, les gaz de combustion de mélanges d'air et d'hydrocarbures de richesse supérieure à 1 et de leurs mélanges. De préférence, le milieu réducteur contient de l'hydrogène. De manière très préférée, ladite étape c) est réalisée en présence d'hydrogène à une température comprise entre 100 et 550°C. Il est avantageux de mélanger lesdits composés réducteurs à un composé inerte tel que par exemple, l'azote ou l'argon, qui ne modifie pas le caractère réducteur des composés réducteurs, précédemment cités. Pour la mise en oeuvre de ladite étape c), le solide issu de ladite étape b) est généralement introduit dans un montage en verre comportant un tube qui se glisse dans un four vertical. Ce tube est équipé d'un fritté qui supporte ledit solide comprenant le platine sur le support. A la base du tube se trouve un système d'alimentation en différents gaz et d'évacuation de liquides. Au sommet du tube se trouve une ampoule en verre équipée d'un robinet inférieur pour le raccordement au tube et de 3 robinets supérieurs pour la sortie des gaz et l'introduction de réactifs. Ledit solide issu de ladite étape b) est placé dans le tube.

L'étape c) du procédé de préparation du catalyseur selon l'invention est suivie soit d'une étape d1) consistant à imprégner ledit support issu de ladite étape c) par une solution aqueuse soit d'une étape d2) consistant à traiter ledit support, issu de ladite étape c), par au moins un composé donneur d'hydrogène à une température comprise entre 0 et 200 °C, de préférence entre 10 et 50°C. L'ordre de réalisation des étapes d1) et d2) est indifférent dès lors que lesdites étapes sont réalisées à la suite de ladite étape c) et préalablement à l'étape e) décrite dans la suite de la description : soit l'étape d1) précède l'étape d2) soit l'étape d2) précède l'étape d1). Selon l'étape d1), l'imprégnation du support, issu de l'étape c) voire de l'étape d2), est réalisée au moyen d'une solution aqueuse à une température comprise entre 10°C et 100°C, de préférence entre 10 et 40°C. La durée de l'étape d1) est comprise entre 15 et 30 minutes. Le volume de ladite solution aqueuse utilisée pour l'imprégnation selon l'étape d1) est supérieur ou égal au volume poreux du support additionné du volume intergranulaire de sorte que le lit de catalyseur est recouvert de solution. Le volume de solution est généralement compris entre 0,5 et 50 ml par g de solide, de préférence entre 1 et 20 ml par g de solide. La solution aqueuse peut être constituée uniquement d'eau ou comprendre de l'eau et un agent compétiteur tel que le chlore, l'agent compétiteur, préférentiellement le chlore, ayant une concentration comprise entre 0,01 et 1 % dans ladite solution aqueuse. L'excédent de solution aqueuse est éliminé par écoulement, la solution excédentaire étant recueillie dans un réceptacle. Lorsque l'étape d1) précède l'étape d2), l'excédent de ladite solution aqueuse peut être éliminé soit avant soit immédiatement après l'étape d2). Conformément à l'étape d2), ledit composé donneur d'hydrogène est avantageusement choisi parmi l'hydrogène, l'ammoniac, les hydrocarbures aliphatiques, saturés ou insaturés et les hydrocarbures aromatiques, de préférence il s'agit de l'hydrogène. Ladite étape d2) est réalisée préférentiellement à une température comprise entre 10 et 50°C. Elle dure entre 5 et 60 minutes et de préférence entre 10 et 30 minutes. Lorsque l'étape d1) précède ladite étape d2), le support issu de l'étape c) est ramené à température ambiante et avantageusement soumis à un balayage par un gaz neutre, par exemple l'azote, avant la mise en oeuvre de ladite étape d1) elle-même. Selon l'étape e) du procédé de préparation du catalyseur selon l'invention, ledit support poreux, déjà imprégné par du platine au cours de ladite étape a), est imprégné par au moins une solution contenant au moins un précurseur de rhodium. Le volume de solution contenant au moins ledit précurseur de rhodium est avantageusement compris entre le dixième et cent fois le volume poreux dudit support, de préférence entre le cinquième et dix fois le volume poreux dudit support. Pour la mise en oeuvre de ladite étape e), le précurseur de rhodium est choisi parmi les composés solubles en milieu inorganique ou en milieu organique. Le précurseur de rhodium est notamment choisi parmi le chlorure de rhodium, le nitrate de rhodium, le chlorure de chloro-rhodium pentammine, l'acétylacétonate de rhodium. La concentration du précurseur de rhodium, dans la solution est choisie telle que, à l'issue du procédé de préparation du catalyseur selon l'invention, la concentration en rhodium, dans ledit catalyseur, exprimée en poids par rapport audit catalyseur, est comprise entre 0,05 et 3 %, de préférence entre 0,1 et 1 % et très préférentiellement entre 0,2 et 0,6 %. La solution contenant le précurseur de rhodium, peut, si nécessaire, contenir des ions hydrogène, ammonium, chlorures, nitrates. La température d'imprégnation du précurseur de rhodium, est en général comprise entre 0 et 100°C, de préférence entre 10 et 40°C. La durée d'imprégnation est en général comprise entre 1 minute et 10 heures, de préférence entre 2 minutes et 4 heures.

L'imprégnation du précurseur de rhodium, est effectuée sous atmosphère neutre ou oxydante voire réductrice. L'étape e) est préférentiellement réalisée sous une atmosphère gazeuse dépourvue d'hydrogène.

La préparation du catalyseur selon l'invention se termine par une étape f) consistant à effectuer une activation du support poreux, imprégnés de platine et de rhodium, en atmosphère neutre, réductrice ou oxydante à une température comprise entre 100 et 800°C, de préférence entre 300 et 600°C, éventuellement suivie d'une activation dans un milieu réducteur à une température comprise entre 0 et 800°C, de préférence entre 100 et 500°C.

Afin d'augmenter la teneur en rhodium et d'en contrôler l'ajout dans le catalyseur, l'étape e) est avantageusement immédiatement suivie d'une étape e1) comprenant au moins le balayage du catalyseur, issu de ladite étape e), sous hydrogène suivi d'une nouvelle imprégnation dudit catalyseur par une solution contenant au moins un précurseur de rhodium. L'étape e1) est reproduite jusqu'à l'obtention de la teneur en rhodium, voulue dans le catalyseur selon l'invention.

La présente invention a également pour objet l'utilisation du catalyseur selon l'invention dans un procédé pour l'ouverture sélective d'au moins un composé naphténique choisi parmi le méthylcyclopentane, le méthylcyclohexane et leur mélange.

Selon l'invention, il est recherché, par le procédé d'ouverture sélective du méthylcyclopentane et/ou du méthylcyclohexane selon l'invention, la production de paraffines, préférentiellement monobranchées. L'ouverture sélective du méthylcyclopentane et/ou du méthylcyclohexane, réalisée selon le procédé de l'invention, nécessite la coupure d'une seule liaison carbone-carbone du méthylcyclopentane et/ou du méthylcyclohexane. L'ouverture sélective du méthylcyclopentane conduit au 2-méthylpentane, au 3-méthylpentane et/ou au n-hexane. L'ouverture sélective du méthylcyclohexane conduit au 2-méthylhexane, au 3-méthylhexane et/ou au n-heptane. Des réactions secondaires indésirables liées à la déshydrogénation du méthylcyclopentane et/ou du méthylcyclohexane, à l'isomérisation des paraffines linéaires et monobranchées produites et également au craquage du méthylcyclopentane et/ou du méthylcyclohexane peuvent être observées et doivent être limitées au maximum afin de favoriser la réaction principale, à savoir celle de l'ouverture sélective du méthylcyclopentane et/ou du méthylcyclohexane.

Le procédé d'ouverture sélective du méthylcyclopentane et/ou du méthylcyclohexane selon l'invention est généralement opéré dans les conditions suivantes : une température comprise entre 200 et 500°C, de préférence entre 250 et 400°C, une pression totale comprise entre 2 et 5 MPa, de préférence entre 2,5 et 4 MPa, un rapport molaire H₂/composé(s) naphténique(s) compris entre 3 et 8 et une ppH (débit massique horaire de charge / masse du catalyseur) comprise entre 2 et 20 h⁻¹, de préférence entre 2 et 10 h⁻¹.

Les exemples qui suivent illustrent l'invention.

### Exemple 1 (invention) : Préparation d'un catalyseur comprenant du platine et du rhodium.

a) Dix grammes d'un support alumine gamma ayant une surface spécifique de 215 m²/g et un volume poreux de 0,6 cm³/g et se présentant sous forme de billes de 2 mm de diamètre sont imprégnés sous agitation dans un bécher par 15 cm³ d'une solution d'acide chlorhydrique à 1 mol/l. Après 15 min, 50 cm³ d'une solution d'acide chloroplatinique contenant 0,12 g de platine sont ajoutés sous agitation. Après une heure de contact avec la solution contenant l'acide chloroplatinique, le bécher est mis sur un bain de sable à 60°C et maintenu sous agitation jusqu'à évaporation de la solution. Le bécher est alors placé dans une étuve à 120°C pendant 16 heures.
b) L'alumine imprégnée par l'acide chloroplatinique est introduite dans un four tubulaire et subit un traitement de calcination sous air à un débit de 10 L/h avec une montée en température de l'ambiante à 10°C/min jusqu'à 450°C suivie d'un palier de 4 heures à 450°C. Il en résulte un solide calciné comprenant du platine sur alumine.
c) Ledit solide calciné est introduit dans un montage en verre comportant un tube qui se glisse dans un four vertical. Ce tube est équipé d'un fritté qui supporte ledit solide. A la base du tube se trouve un système d'alimentation en différents gaz et d'évacuation de liquides. Au sommet du tube se trouve une ampoule en verre équipée d'un robinet inférieur pour le raccordement au tube et de 3 robinets supérieurs pour la sortie des gaz et l'introduction de réactifs.

Le solide calciné platine sur alumine est placé dans le tube puis réduit sous hydrogène (10 L/h) lors d'une montée en température à 10°C/min de la température ambiante jusqu'à 500°C suivi d'un palier à 500°C pendant une heure puis d'un retour à la température ambiante.
d1) Le solide est ensuite balayé par un flux d'azote (10 L/h) pendant 15 min puis il est mouillé par 15 cm³ d'eau pure désaérée introduite à partir de l'ampoule en verre. L'excédent de solution aqueuse est éliminé par écoulement.
d2) Le solide est ensuite balayé à température ambiante par un flux d'hydrogène de 10 L/h pendant 15 min puis un flux d'azote de 10 L/h pendant 15 min.
e) Deux cent ml d'une solution de nitrate de rhodium contenant 0,04 g de rhodium sont introduits dans ampoule puis purgée sous azote pendant 10 min. La solution est ensuite versée sur le solide comprenant le platine sur alumine. L'ensemble solide-solution est agité par un contre-courant d'azote (2 L/h) pendant 5 min. Le débit d'azote est ensuite augmenté jusqu'à 10 L/h à la température ambiante et le catalyseur séché dans ces conditions pendant 45 min.
f) Le flux d'azote est remplacé par de l'hydrogène (10 L/h) et la température est portée à 100°C. Après 12 h, la température du catalyseur est portée à 500°C à la vitesse de 2°C/min puis maintenue à cette valeur pendant une heure.

Le catalyseur C1 ainsi préparé contient outre l'alumine, 1,17 % poids de platine, 0,39 % poids de rhodium et 1,4 % poids de chlore.

### Exemple 2 (comparatif) : Préparation d'un catalyseur comprenant du platine et du rhodium.

a) Dix grammes d'un support alumine gamma ayant une surface spécifique de 215 m²/g et un volume poreux de 0,6 cm³/g et se présentant sous forme de billes de 2 mm de diamètre sont imprégnés sous agitation dans un bécher par 15 cm³ d'une solution d'acide chlorhydrique de concentration égale à 1 mol/l. Après 15 min, 50 cm³ d'une solution d'acide chloroplatinique contenant 0,12 g de platine sont ajoutés sous agitation. Après une heure de contact avec la solution contenant l'acide chloroplatinique, le bécher est mis sur un bain de sable à 60°C et maintenu sous agitation jusqu'à évaporation de la solution. Le bécher est alors placé dans une étuve à 120°C pendant 16 heures.
b) L'alumine imprégnée par l'acide chloroplatinique est introduite dans un four tubulaire et subit un traitement de calcination sous air à un débit de 10 L/h avec une montée en température de l'ambiante à 10°C/min jusqu'à 450°C suivie d'un palier de 4 heures à 450°C. Il en résulte un solide calciné comprenant du platine sur alumine.
c) Ledit solide calciné est introduit dans un montage en verre comportant un tube qui se glisse dans un four vertical. Ce tube est équipé d'un fritté qui supporte ledit solide. A la base du tube se trouve un système d'alimentation en différents gaz et d'évacuation de liquides. Au sommet du tube se trouve une ampoule en verre équipée d'un robinet inférieur pour le raccordement au tube et de 3 robinets supérieurs pour la sortie des gaz et l'introduction de réactifs.

Le solide comprenant du platine sur alumine est placé dans le tube puis réduit sous hydrogène (10 L/h) lors d'une montée en température à 10°C/min de la température ambiante jusqu'à 500°C suivi d'un palier à 500°C pendant une heure puis d'un retour à la température ambiante.
d2) Ledit solide est ensuite balayé à température ambiante par un flux d'hydrogène de 10 L/h pendant 15 min puis un flux d'azote de 10 L/h pendant 15 min.
e) Deux cent mL d'une solution de nitrate de rhodium contenant 0,04 g de rhodium sont introduits dans l'ampoule puis purgée sous azote pendant 10 min. La solution est ensuite versée sur ledit solide comprenant le platine sur alumine. L'ensemble solide-solution est agité par un contre-courant d'azote (2 L/h) pendant 5 min. Le débit d'azote est ensuite augmenté jusqu'à 10 L/h à la température ambiante et le catalyseur séché dans ces conditions pendant 45 min.
f) Le flux d'azote est remplacé par de l'hydrogène (10 L/h) et la température est portée à 100°C. Après 12 h, la température du catalyseur est portée à 500°C à la vitesse de 2°C/min puis maintenue à cette valeur pendant une heure.

Le catalyseur C2 ainsi préparé contient outre l'alumine, 1,17 % poids de platine, 0,39 % poids de rhodium et 1,4 % poids de chlore.

### Exemple 3 (comparatif): préparation d'un catalyseur à base d'iridium.

a) Dix grammes d'un support alumine gamma ayant une surface de 215 m²/g et un volume poreux de 0,6 cm³/g et se présentant sous forme de billes de 2 mm de diamètre sont imprégnés sous agitation dans un bécher par 15 cm³ d'une solution d'acide chlorhydrique de pH égal à 1. Après 15 min, 50 cm³ d'une solution d'acide chloroiridique contenant 0,16 g d'iridium sont ajoutés sous agitation. Après une heure de contact avec la solution contenant l'acide chloroiridique, le bécher est mis sur un bain de sable à 60°C et maintenu sous agitation jusqu'à évaporation de la solution. Le bécher est alors placé dans une étuve à 120°C pendant 16 heures.
b) L'alumine imprégnée par l'acide chloroiridique est introduite dans un four tubulaire et subit un traitement de calcination sous air à un débit de 10 Uh et par heure avec une montée en température de l'ambiante à 10°C/min jusqu'à 300°C suivie d'un palier de 4 heures à 300°C puis d'un refroidissement à la température ambiante. Il en résulte un catalyseur iridium sur alumine calciné.
c) Le catalyseur calciné est ensuite réduit sous hydrogène (10 L/h) avec montée en température à 2°C/min jusqu'à 500°C puis palier de température à 500°C pendant 4 h.

Le catalyseur C3 ainsi préparé contient outre l'alumine, 1,56 % poids d'iridium et 1,47 % poids de chlore.

### Exemple 4 : Utilisation des catalyseurs C1, C2 et C3 en ouverture du méthylcyclohexane et/ou du méthylcyclopentane.

Les catalyseurs des exemples 1 à 3 (C1, C2 et C3) sont testés successivement pour l'ouverture de cycle du méthylcyclohexane, du méthylcyclopentane et du mélange méthylcyclohexane-méthylcyclopentane dans une installation comportant :
- un circuit d'alimentation en hydrogène permettant de réguler le débit et la pression via un débitmètre Brooks 5866,
- un circuit d'alimentation en charge liquide à débit contrôlé par une pompe piston,
- un réacteur métallique placé dans un four,
- un régulateur de pression,
- un séparateur gaz-liquide placé sur le circuit effluents du réacteur,
- un chromatographe Varian 3400 équipé d'une colonne alumina PLOT pour séparer les produits d'ouverture de cycle, de craquage, d'isomérisation et de déshydrogénation.

Préalablement à chacun des tests, chacun des catalyseurs C1 à C3 est broyé à une granulométrie comprise entre 200 et 400 microns. Chacun des catalyseurs C1 à C3 est ensuite chargé (250 mg) dans le réacteur entre deux lits de carborundum pour améliorer l'hydrodynamique du lit catalytique.

Dans une première étape, chacun des catalyseurs C1 à C3 est réduit une heure à 500°C sous hydrogène (40 bars, 3,6 L/h). La température est ramenée à 200°C et la charge liquide (méthylcyclohexane, méthylcyclopentane ou mélange 50-50 en masse méthylcyclohexane-méthylcyclopentane) est injectée à une PPH de 2,5 (2,5 g/g de catalyseur/h) sous une pression totale de 3 MPa. Le débit molaire H₂/hydrocarbure(s) est fixé à 5.

L'analyse chromatographique des produits de la réaction montre qu'un palier de transformation est atteint au bout de 2 h. La température est alors augmentée de 25°C en 2 h puis un palier de 2 h est réalisé. Ce cycle montée en température suivi d'un palier est poursuivi tous les 25°C jusqu'à 400°C.

Le tableau ci-dessous compare les rendements maximums en ouverture de cycle pour les tests réalisés avec le méthylcyclohexane (MCH) pur, le méthylcyclopentane (MCP) pur et le mélange méthylcyclohexane-méthylcyclopentane (MCH-MCP) :

| | MCH | MCP | MCH-MCP |
|---|---|---|---|
| catalyseur C1 | 55% | 41 | 44 |
| catalyseur C2 | 53% | 39 | 42 |
| catalyseur C3 | 42 % | 37 | 39 |

Le catalyseur C1 selon l'invention conduit à un rendement maximal en produits d'ouverture de cycle nettement plus élevé que le catalyseur 3 et toujours sensiblement plus élevé que le catalyseur 2.

## Revendications

1. Catalyseur comprenant au moins un support poreux, au moins du platine et au moins du rhodium, ledit catalyseur ayant été préparé selon un procédé comprenant au moins:
a) l'imprégnation du support par au moins une solution contenant un précurseur de platine,
b) l'activation sous atmosphère neutre ou oxydante à une température comprise entre 120 et 800°C,
c) l'activation en milieu réducteur à une température comprise entre 0 et 800°C,
d1) l'imprégnation par une solution aqueuse et d2) le traitement par au moins un composé donneur d'hydrogène à une température inférieure à 200 °C, l'ordre de réalisation des étapes d1) et d2) est indifférent dès lors que lesdites étapes sont réalisées à la suite de ladite étape c) et préalablement à l'étape e) qui suit,
e) l'imprégnation du support, déjà imprégné par du platine, par au moins une solution contenant un précurseur de rhodium,
f) l'activation sous atmosphère neutre, réductrice ou oxydante à une température comprise entre 100 et 800°C.

2. Catalyseur selon la revendication 1 tel que ledit support poreux est choisi parmi les alumines et les alumines chlorées.

3. Catalyseur selon la revendication 1 ou la revendication 2 tel qu'il comprend du chlore.

4. Catalyseur selon l'une des revendications 1 à 3 tel que ledit précurseur de platine utilisé dans ladite étape a) est l'acide chloroplatinique.

5. Catalyseur selon l'une des revendications 1 à 4 tel que ladite étape b) est réalisée à une température comprise entre 300 et 600°C.

6. Catalyseur selon l'une des revendications 1 à 5 tel que ladite étape c) est réalisée en présence d'hydrogène à une température comprise entre 100 et 550°C.

7. Catalyseur selon l'une des revendications 1 à 6 tel que ladite solution aqueuse utilisée selon l'étape d1) est soit constituée uniquement d'eau soit comprend de l'eau et un agent compétiteur.

8. Catalyseur selon l'une des revendications 1 à 7 tel que ledit composé donneur d'hydrogène utilisé dans ladite étape d2) est l'hydrogène.

9. Catalyseur selon l'une des revendications 1 à 8 tel que ladite activation en atmosphère neutre ou oxydante selon ladite étape est suivie d'une activation dans un milieu réducteur à une température comprise entre 0 et 800°C.

10. Catalyseur selon l'une des revendications 1 à 9 tel qu'il est préparé selon un procédé comprenant au moins une étape e1) comprenant au moins le balayage du catalyseur, issu de ladite étape e), sous hydrogène suivi d'une nouvelle imprégnation dudit catalyseur par une solution contenant au moins un précurseur de rhodium.

11. Procédé pour l'ouverture sélective d'au moins un composé naphténique choisi parmi le méthylcyclopentane, le méthylcyclohexane et leur mélange réalisé en présence d'un catalyseur selon l'une des revendications 1 à 10, ledit procédé étant réalisé à une température comprise entre 200 et 500°C, une pression totale comprise entre 2 et 5 MPa, un rapport molaire H₂/composé(s) naphténique(s) compris entre 3 et 8 et une ppH (débit massique horaire de charge / masse du catalyseur) comprise entre 2 et 20 h⁻¹.

## Claims

1. A catalyst comprising at least one porous support, at least some platinum, and at least some rhodium, said catalyst having been prepared by way of a method comprising, at least:
a) impregnation of the support with at least one solution containing a platinum precursor,
b) activation in a neutral or oxidising atmosphere, at a temperature of between 120 and 800°C,
c) activation in a reducing medium, at a temperature of between 0 and 800°C,
d1) impregnation with an aqueous solution and d2) treatment with at least one hydrogen donor compound at a temperature of less than 200°C, the order in which steps d1) and d2) are carried out not mattering, provided that said steps arc carried out after said step c) and before step e) which follows,
e) impregnation of the support, having already been impregnated with platinum, by means of at least one solution containing a rhodium precursor,
f) activation in a neutral, reducing or oxidising atmosphere, at a temperature of between 100 and 800°C.

2. A catalyst according to Claim 1, such that said porous support is selected from aluminas and chlorinated aluminas.

3. A catalyst according to Claim 1 or Claim 2, such that it comprises chlorine.

4. A catalyst according to one of Claims 1 to 3, such that said platinum precursor which is used in said step a) is chloroplatinic acid.

5. A catalyst according to one of Claims 1 to 4, such that said step b) is carried out at a temperature of between 300 and 600"C.

6. A catalyst according to one of Claims 1 to 5, such that said step c) is carried out in the presence of hydrogen, at a temperature of between 100 and 550°C.

7. A catalyst according to one of Claims 1 to 6, such that said aqueous solution which is used in accordance with step d1) is either constituted by water alone, or comprises water and a competitor agent.

8. A catalyst according to one of Claims 1 to 7, such that said hydrogen donor compound which is used in step d2) is hydrogen.

9. A catalyst according to one of Claims 1 to 8, such that said activation in a neutral or oxidising atmosphere in accordance with step f) is followed by activation in a reducing medium at a temperature of between 0 and 800°C.

10. A catalyst according to one of Claims 1 to 9, such that it is prepared in accordance with a process comprising at least step e1), comprising, at least, scavenging of the catalyst resulting from said step e), in hydrogen, followed by renewed impregnation of said catalyst with a solution containing at least a rhodium precursor.

11. A process for the selective opening of at least one naphthenic compound selected from methylcyclopentane, methylcycohexane, and a mixture thereof carried out in the presence of a catalyst according to one of Claims 1 to 10, said process being carried out at a temperature of between 200 and 500°C, a total pressure of between 2 and 5 MPa, a H₂/naphthenic compound(s) molar ratio of between 3 and 8, and a ppH (hourly mass flow rate of charge / mass of catalyst) of between 2 and 20 h⁻¹.

## Patentansprüche

1. Katalysator, umfassend mindestens einen porösen Träger, mindestens Platin und mindestens Rhodium, wobei der Katalysator gemäß einem Verfahren hergestellt wurde, das mindestens Folgendes umfasst:
a) Durchtränken des Trägers mit mindestens einer Lösung, die eine Platin-Vorläufersubstanz enthält,
b) Aktivieren in einer neutralen oder oxidierenden Atmosphäre, bei einer Temperatur im Bereich von 120 bis 800°C,
c) Aktivieren in einem reduzierenden Milieu, bei einer Temperatur im Bereich von 0 bis 800 °C,
d1) Durchtränken mit einer wässrigen Lösung und
d2) Behandeln mit mindestens einer Verbindung, die bei einer Temperatur von weniger als 200 °C Wasserstoff abgibt, wobei die Reihenfolge der Durchführung der Schritte d1) und d2) beliebig ist, solange diese Schritte nach dem Schritt c) und vor dem nachfolgenden Schritt e) durchgeführt werden,
e) Durchtränken des Trägers, der bereits mit Platin durchtränkt ist, mit mindestens einer Lösung, die eine Rhodium-Vorläufersubstanz enthält,
f) Aktivieren in einer neutralen, reduzierenden oder oxidierenden Atmosphäre, bei einer Temperatur im Bereich von 100 bis 800 °C,

2. Katalysator nach Anspruch 1, derart, dass der poröse Träger aus den Aluminiumoxiden und den chlorierten Aluminiumoxiden ausgewählt ist.

3. Katalysator nach Anspruch 1 oder Anspruch 2, derart, dass er Chlor umfasst.

4. Katalysator nach einem der Ansprüche 1 bis 3, derart, dass er sich bei der Platin-Vorläufersubstanz, die in Schritt a) verwendet wird, um Chlorplatinsäure handelt.

5. Katalysator nach einem der Ansprüche 1 bis 4, derart, dass der Schritt b) bei einer Temperatur im Bereich von 300 bis 600 °C durchgeführt wird.

6. Katalysator nach einem der Ansprüche 1 bis 5, derart, dass der Schritt c) in Gegenwart von Wasserstoff bei einer Temperatur im Bereich von 100 bis 550 °C durchgeführt wird.

7. Katalysator nach einem der Ansprüche 1 bis 6, derart, dass die wässrige Lösung, die gemäß Schritt d1) verwendet wird, entweder ausschließlich aus Wasser besteht oder Wasser und ein konkurrierendes Mittel umfasst.

8. Katalysator nach einem der Ansprüche 1 bis 7, derart, dass es sich bei der Verbindung, die Wasserstoff abgibt und in Schritt d2) verwendet wird, um Wasserstoff handelt.

9. Katalysator nach einem der Ansprüche 1 bis 8, derart, dass im Anschluss an die Aktivierung in neutraler oder oxidierender Atmosphäre gemäß Schritt f) eine Aktivierung in einem reduzierenden Milieu bei einer Temperatur im Bereich von 0 bis 800 °C erfolgt.

10. Katalysator nach einem der Ansprüche 1 bis 9, derart, dass er gemäß einem Verfahren hergestellt ist, das mindestens einen Schritt a1) umfasst, welcher mindestens das Spülen des Katalysator, der in Schritt e) erhalten wurde, unter Wasserstoff umfasst, woraufhin ein erneutes Durchtränken des Katalysators mit einer Lösung, die eine mindestens eine Rhodium-Vorläufersubstanz enthält, erfolgt.

11. Verfahren zur selektiven Öffnung mindestens einer gesättigten alicyclischen Verbindung, die aus Methylcyclopentan, Methylcyclohexan und deren Mischung ausgewählt ist, durchgeführt in Gegenwart eines Katalysators nach einem der Ansprüche 1 bis 10, wobei das Verfahren bei einer Temperatur im Bereich von 200 bis 500 °C, einem Gesamtdruck im Bereich von 2 bis 5 MPa, einem Molverhältnis von H₂ / gesättigte alicyclische Verbindung(en) im Bereich von 3 bis 8 und einem ppH (massenbezogener Stundendurchsatz der Charge / Masse des Katalysators) im Bereich von 2 bis 20 h⁻¹ durchgeführt wird.
